# EUROPEAN PATENT APPLICATION

(11) **EP 3 354 304 A1**
(43) Date of publication of application: **01.08.2018**
(21) Application number: 17305101.2
(22) Date of filing: 30.01.2017
(51) Int. Cl.: A61M 5/142, A61M 5/145, A61M 5/148, A61J 1/10

(54) **DEVICE FOR PROPER DRAINING OF AN INFUSION BAG UNDER PRESSURE**

(71) Applicant: Guerbet, 93420 Villepinte (FR)
(72) Inventor: DAVAL, Jean, 69100 VILLEURBANNE (FR); BEN ABDESSLEM, Yassine, 69003 LYON (FR)
(74) Representative: Regimbeau

(57) **Abstract**

The invention relates to a device for proper draining of an infusion bag under pressure, the device comprising a casing accommodating at least a deformable cushion and a variable volume bladder, wherein said casing comprises a first casing portion (14) defining a cavity (15) having a depth, said deformable cushion being arranged on a surface of said cavity (15) over a cushion thickness, said first casing portion (14) comprising an output side (18) configured to accommodate an output tube of the bag,
the first casing portion (14) comprises an elevated part (22) protruding from the cavity (15) and extending from the output side (18) towards an opposite side (19) of the cavity, the deformable cushion covering the cavity and the elevated part, said elevated part defining a support area for the bag.

## Description

### TECHNICAL FIELD

The present invention concerns the general technical field of product administration to a patient, in particular parental administration such as intravenous injection.

### GENERAL DESCRIPTION OF THE PRIOR ART

There are known devices for injecting a liquid solution containing a product such as a contrast agent into a patient, for example for medical imaging, which encompasses X-ray imaging and magnetic resonance imaging. Some of these devices use a flexible bag containing the liquid solution to be injected to the patient.

Such a bag conventionally comprises two superposed plastic sheets welded at their peripheries for defining a volume intended to contain the solution containing the product to be injected, and an output tube sealed between the superposed sheets in order to define a circulation channel for the solution between the inside and the outside of the bag.

The administration of the product to patient requires the product be emptied out of the bag, which may be performed through gravity or with a device applying a force on the bag. For example, as depicted on figures 1 to 3b, patent application WO 2013/186289 describes a device 1 for the injection of a liquid product 2 contained in a flexible bag 3, said device comprising at least a constant volume deformable cushion 4 and a variable volume bladder 5 arranged in a casing 6.

A connector 7 is fixed at the free extremity of the tube to allow the bag being connected to a delivering assembly connected to the patient. A hydraulic feeding port 8 feeds the variable volume bladder 5 with hydraulic fluid from a hydraulic source.

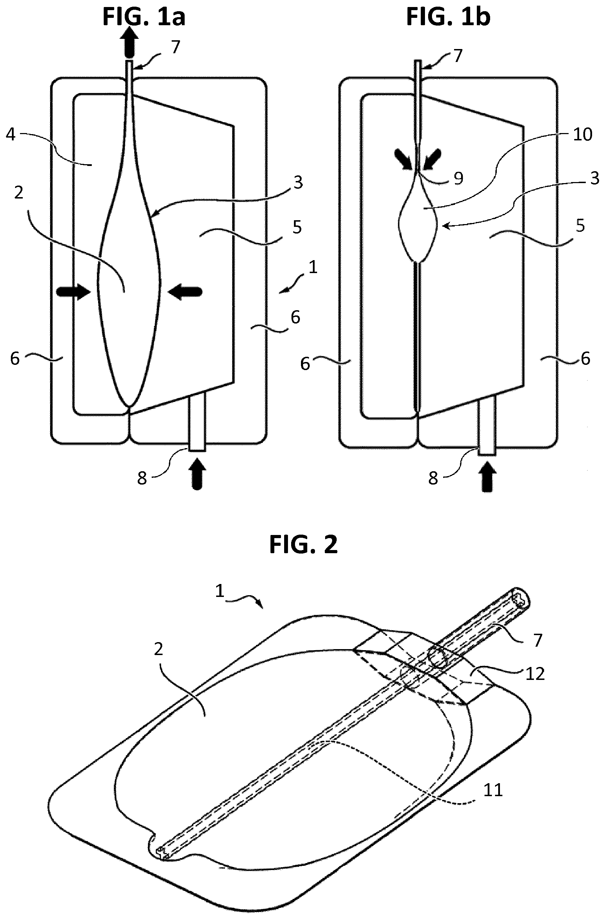

During the draining of the bag, the hydraulic fluid contained in the bladder 5 pressures one wall of the bag 3 toward the other in order to eject the solution 2 out of the bag 3. The pressure applied on the bag may generate a collapsing point 9 of the superposed sheets forming the walls of the bag 3, as illustrated on Figure 1 b.

At this collapsing point 9, the walls of the bag 3 are pressed one onto the other, and are being clung together. The collapsed walls block the circulating channel of the solution towards the output tube 7. A residual volume 10 of the solution 2 is therefore prevented from leaving the bag 3. This collapsing of the walls usually appears at the end of the injection process, when the solution to be injected is concentrated on the center of the bag 3: the lateral pressure applied by the hydraulic fluid blocks the solution path at the upper part of the bag, forming a watertight obstruction.

This residual volume 10 may represent a significant amount of the volume of the solution 2 originally contained in the bag 3 (up to 20% of the solution, or the equivalent of a dose for a patient). The product contained in the solution may be onerous, as often with contrast products. Avoiding wasting the solution may therefore be desirable. In addition, when this phenomenon occurs during an injection, the injection process may have to be restarted, because the patient might have not received a sufficient amount of product. In more than 95% of the uses of prior art device there is no problem. But when the bag is pressed at a high pressure and/or when the catheter which is connected to the bag 3 is very small and/or when it remains in the bag 3 a volume lower than the volume which has to be injected, this phenomenon can occur.

The global time of injection is an important parameter for a radiologist. Indeed, a radiologist needs to know with a good precision the duration of the injection in order to synchronize the scanner with the injection of contrast agent. By the use of a prior art device and when the phenomenon occurs, the end of the injection could be substantially delayed. This can lead to a failure of the X ray examination. A failed X ray examination will induce a new examination with the injection of an additional dose of X-rays and an additional dose of contrast agent.

As illustrated by Figure 2, patent application WO 2015/121355 proposes arranging a spacer member 11 extending inside the bag 3 between the superposed sheets in order to keep a channel open for the flow of the solution. The spacer member 11 might be received in the pipe of rigid access member 12 accommodating the output tube 7.

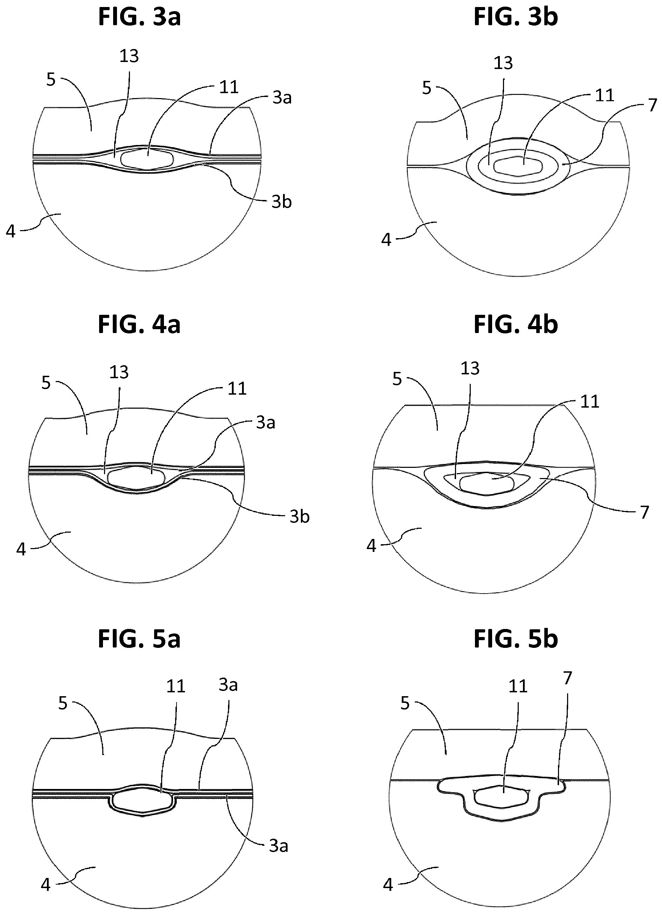

The spacer member 11 reduces the likelihood of a collapsing of the walls, but fails to avoid any risk of it, especially under a high pressure. An example is shown in figures 3a, 3b, 4a, 4c, 5a, 5c. Figures 3a and 3b show a bag portion section and a tube portion section, respectively, at the beginning of the injection process. The bladder 5 pressures a wall 3a, 3b of the bag 3 with a low pressure. Both the bladder 5 and the deformable cushion 4 deform around the bag 3, and the two walls 3a, 3b of the bag come close to one another, but channels 13 on either side of the spacer member 11 are kept open allow the solution to flow towards the output tube 7 of the bag 3. Since the pressure is low, the tube 7, more rigid than the bag 3, keep an oval shape round the spacer, with space forming channel 13 all around it.

Figures 4a and 4b show a bag portion section and a tube portion section, respectively, later in the injection process, as the pressure increased, the bladder 5 tends to push back the wall 3a of the bag 3 towards the deformable cushion 4. The spacer member 11 however keeps apart the walls 3a, 3b of the bag 3. Channels 13 on either side of the spacer member 11 are thus kept open and allow the solution to flow towards the output tube 7 of the bag 3. The tube 7 is deformed, and contacts the spacer 11 in front of both the bladder 5 and the deformable cushion 4, but keeps channels 13 on either side of the spacer member 11 for the solution to flow.

Figures 5a and 5b show a bag portion section and a tube portion section, respectively, with a higher pressure is applied to the bladder 5. Sometimes, because of the high pressures (up to 15-21 bars); the deformable cushion 4 would completely fit the shape of the spacer member 11, pressing the wall 3b of the bag 3 against the spacer member 11. The walls 3a, 3b of the bag 3 block any possible channel, resulting in a collapsing point. Compared to the configuration illustrated on Figures 3a or 4a, there is no channel left for the solution to flow outside the bag 3. This may also happen to the tube portion section, as depicted on Figure 5b. As shown on Figure 1b, the collapsing point 9 may block a residual volume 10 of solution, which cannot leave the bag 3.

It shall be noted that the deformable cushion must be made of a material with high elongation properties, for example up to 750 % tensile elongation at break, in order for the deformable cushion to mold the bag so as to guarantee the best emptying of the bag. The drawback of such a high elongation capability is that collapsing may sometimes occurs, as explained above.

As a result, there is a need for a device allowing a proper draining of an infusion bag under pressure containing a solution, that avoids any residual volume of solution from being left in the bag.

### DESCRIPTION OF THE INVENTION

To this end, the invention proposes a device for draining a bag containing a solution, the device comprising a casing accommodating at least a deformable cushion and a variable volume bladder, the device being configured to accommodate the bag between said constant volume deformable cushion and said variable volume bladder,
wherein said casing comprises a first casing portion defining a cavity having a depth, said deformable cushion being arranged on a surface of said cavity over a cushion thickness, said first casing portion comprising an output side configured to accommodate an output tube of the bag,
wherein the first casing portion comprises an elevated part protruding from the cavity and extending from the output side towards an opposite side of the cavity, the deformable cushion covering the cavity and the elevated part, said elevated part defining a support area for the bag.

Other preferred, although non limitative, aspects of the device of the invention are as follows, isolated or in a technically feasible combination:
- the elevated part extends over at least one tenth of a distance between the output side and the opposite side of the cavity;
- the elevated part extends over at least 2 cm from the output side towards the opposite side of the cavity;
- the elevated part protrudes from a deepest part of the cavity of more than half the cavity depth at the deepest part;
- the deformable cushion is arranged on the surface of the cavity and of the elevated part;
- the cushion thickness on the elevated part is less than 8 mm;
- the cushion thickness over a deepest part of the cavity is more than 1 cm;
- the deformable cushion fills the cavity and in the absence of a bag defines a plane covering said cavity and the elevated part;
- the deformable cushion is made of silicone;
- the casing comprises at least a second casing portion, the first casing portion and said second casing portion being articulated in order to enable relative movement of one casing portion relative to the other casing portion between:
   - an open position for putting the bag in place;
   - a closed position for draining the solution contained in the bag.

The invention also relates to a method for draining a solution contained in a bag accommodated between a constant volume deformable cushion and a variable volume bladder of a device according the invention, wherein the variable volume bladder is deformed by a source of hydraulic power feeding said bladder with hydraulic fluid, pressing the bag against the deformable cushion and causing solution to empty out of the bag.

### DESCRIPTION OF THE FIGURES

Other features and advantages of the invention will emerge from the following description, which is purely illustrative and not limiting on the invention and must be read in conjunction with the appended drawings, in which:
- Figures 1 a and 1 b, already discussed, illustrate a collapsing of the walls of a bag while draining the bag;
- Figure 2, already discussed, illustrates a bag with a spacer member within;
- Figures 3a, 3b, 4a, 4b, 5a, and 5b, already discussed, are cross-section views of different draining configurations of a bag provided with a spacer member;
- Figure 6 shows a first casing portion of a casing according to a possible embodiment of the invention;
- Figure 7a and 7b illustrate cross-section views of a bag portion section and a tube portion section, respectively, in a high pressure draining configuration of a bag provided with a spacer member arranged in a device according to a possible embodiment of the invention;
- Figure 8a illustrates a cross-section view of a first casing portion of a casing for a device according to a possible embodiment of the invention;
- Figure 8b shows an enlarged view of Figure 6a;
- Figure 9a and 9b show test results comparing the draining of a bag with a device of a prior art and a device according to a possible embodiment of the invention.

### DETAILED DESCRIPTION

The invention will now be described in more detail with reference to the figures. In the various figures, equivalent elements bear the same reference numbers.

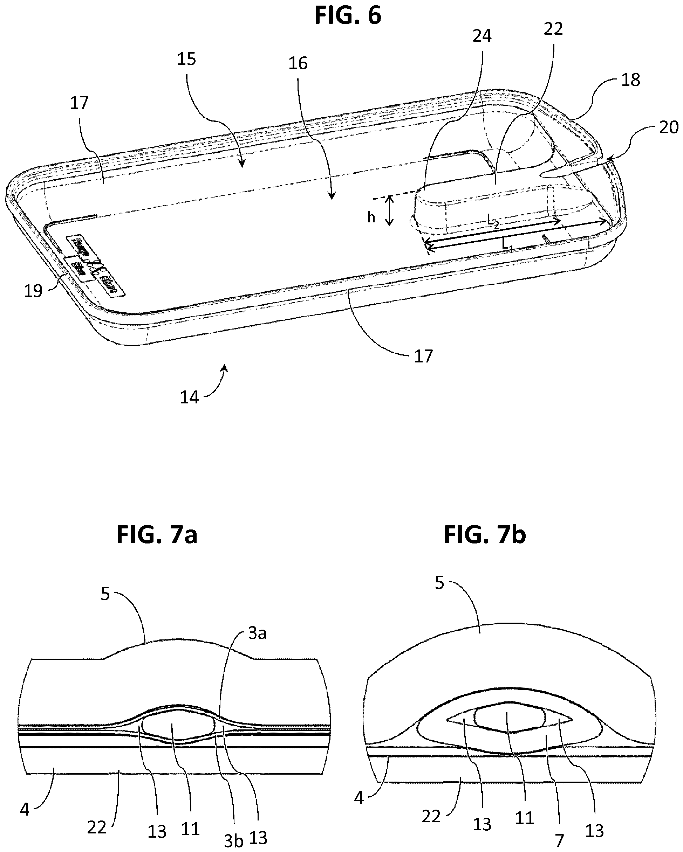

As explained above, a device for draining a bag containing a solution comprises a casing accommodating at least a deformable cushion and a variable volume bladder. The casing comprises a first casing portion for accommodating the deformable cushion. Figure 6 shows such a first casing portion 14.

The first casing portion 14 defines a cavity 15 having a depth. The deformable cushion 4 is to be arranged in the cavity 15, on the surface of said cavity 15. As illustrated, the cavity 15 may be defined by a bottom 16, lateral sides 17, an output side 18 and an opposite side 19 opposite to the output side 18. The output side 18 is configured to accommodate the output tube 7 of the bag 3. In order to do so, the output side 18 comprises a recess 20 constituting a passage for the output tube 7 of the bag 3.

The first casing portion 14 comprises an elevated part 22 protruding from the cavity 15 and extending from the output side 18 towards the opposite side 19 of the cavity 15. The elevated part 22 closes the recess 20. Preferably, the elevated part 22 is integral with the output side 18 of the first casing portion 14. The elevated part 22 may form a peninsula connected to the output side 18 and extending towards a center of the cavity 15.

The elevated part 22 extends towards the opposite side 19 beyond a reception space for the output tube 7 of the bag 3 formed by the recess 20. The elevated part 22 extends over at least one tenth of the distance between the output side 18 and the opposite side 19 of the cavity 15. Preferably, the elevated part extends over a length L₁ of at least 2 cm, preferably of at least 5 cm, more preferably at least 6 cm, from the output side 18 towards the opposite side of the cavity. Preferably, the elevated part extends over a length L₂ of at least 3 cm, preferably of at least 5 cm, more preferably at least 7 cm, from the recess 20 towards the opposite side 19 of the cavity.

The elevated part 22 protrudes from a deepest part of the cavity 15 of more than half the cavity depth at the deepest part. As illustrated, the cavity 15 usually has a flat bottom 16. In this case, the bottom 16 constitutes the deepest part of the cavity. Usually, the elevated part 22 protrudes of at least 1 cm over the bottom of the cavity 15, and more preferably of at least 1,4 cm. The height h of the tip of 24 of the elevated part 22, i.e. the farthest extremity from the output side 18, is thus preferably at least 1 cm over the bottom of the cavity 15, and more preferably of at least 1,4 cm.

The tip 24 of the elevated part 22 preferably has a rounded shape. The top surface of the elevated part 22, i.e. the surface opposing the bottom 16 of the cavity 15, is preferably flat, as depicted.

The deformable cushion 4 fills the cavity 15. Preferably, the deformable cushion 4 is arranged on the surface of the cavity 15 and of the elevated part 22. As shown on Figure 6a, the deformable cushion 4 defines a plane above both the elevated part 22 and the cavity 15 in the absence of a bag 3.

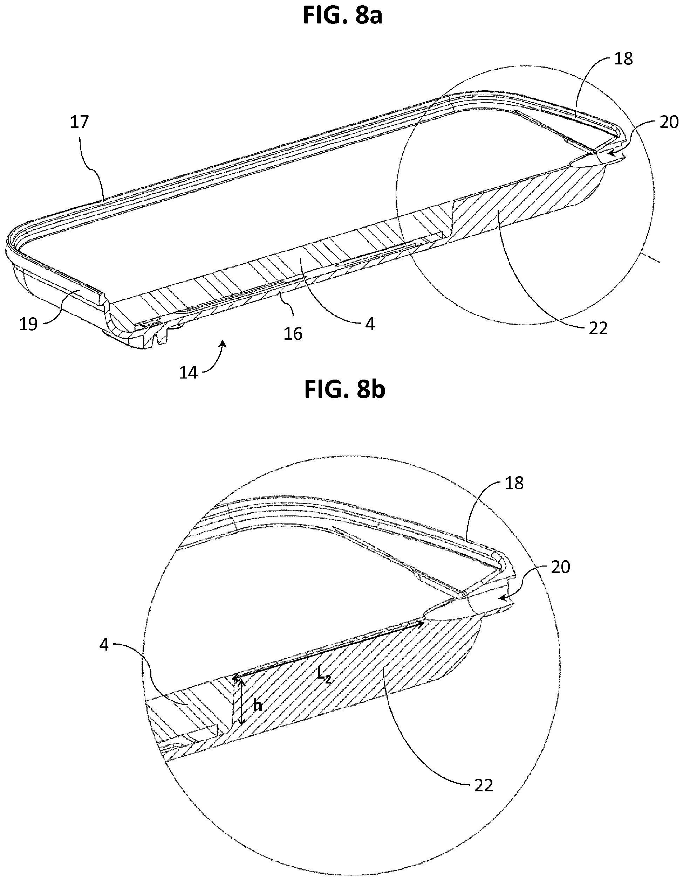

The deformable cushion 4 has a cushion thickness that may be defined as the thickness of the deformable cushion 4 from the surface of the cavity 15. However, the cushion thickness over the peninsula 22 is much lower than the cushion thickness over the bottom 16 of the cavity 15, as shown in figures 8a and 8b. In particular, the cushion thickness on the elevated part 22 is less than 8 mm, preferably less than 4 mm and more preferably less than 1 mm. The cushion thickness may be as small as 0.7 mm. Conversely, the cushion thickness over a deepest part of the cavity 15 is more than 1 cm, preferably more than 1,2 cm, and more preferably more than 1,3 cm.

The deformable cushion 4 is made of a material with a very low hardness, typically a soft polymer. Preferably the soft polymer is a material with a shore hardness of type OO, more preferably of 0 to 80 shore OO and even more preferably of 0 to 50 shore OO. More preferably the deformable cushion 4 is made of a soft polymer which is chosen amongst silicone or polyurethane. Such a polymer is cast into the cavity 15 in a liquid state and solidifies inside the cavity 15. A removable element is arranged in the recess 20 in order to prevent the flow of liquid polymer from filling the recess 20. In a preferred embodiment, the deformable cushion 4 is made of silicone.

The collapsing of the walls of a bag provided with a spacer member requires high pressures. The gravity forcing the solution downwards, the skilled person may have expected that collapsing points would be located at the bottom of the bag 3 when said bag 3 is being emptied out. However, even when the output side of the bag 3 is oriented upwards, the inventor has discovered that the higher pressure areas of the bag are located in the vicinity of the output of the bag 3. The collapsing points are thus likely to be located in the vicinity of the output of the bag 3.

The elevated part 22 acts as a support area for the bag. Since the cushion thickness above the elevated part 22 is low, the bag 3 cannot sink there as much as in the part of the deformable cushion 4 above the bottom 16 of the cavity 15. Figure 7a shows a cross-section view of a bag portion section above the elevated part 22. Figure 7a shows a bag 3 provided with a spacer member 11 and arranged between the deformable cushion 4 and the bladder 5, above the elevated part 22.

The bladder 5 pressures the bag 3. The bladder 5 is deformable and fed with hydraulic fluid from a hydraulic source through a hydraulic feeding port 8. The hydraulic fluid is preferably oil. Filling the bladder with a hydraulic fluid allows to apply variable pressure and to monitor the volume of deformation that is related to the injected volume of contrast agent. Preferably the bladder 5 is made of a material that is much more rigid than silicon. More preferably the bladder 5 is made of rubber. Even more preferably the bladder 5 is made of rubber which is much more rigid than silicone. This difference concerning the rigidity can explain the difference of behavior : the higher the pressure, the more the rubber tends to be flat and the more the silicon will extend to go in every small recess of the bag. When the silicon goes close to the space member, this creates the obstruction. The deformable cushion 4 deforms under the pressure applied to the bag 3. However, the rigid elevated part 22 does not deform and support the bag 3, even under high pressures. The soft material of the deformable cushion 4 cannot fit the shape of the spacer member 11. Channels 13 are kept open in order to allow the flow of the solution. Collapsing of the walls 3a, 3b of the bag 3 is thus prevented.
In a similar way, Figure 7b shows a cross-section view of a tube portion section above the elevated part 22, under pressure. The bladder 5, filled with the hydraulic fluid, pressures the tube 7 against the deformable cushion 4 on the elevated part. The deformable cushion 4 is deformed, but is still supported by the rigid elevated part 22 underneath. As a result, the soft material of the deformable cushion 4 cannot fit the shape of the spacer member 11, and channels 13 inside the deformed tube 7 are kept open on either side of the spacer 11 in order to allow the flow of the solution. Collapsing of the tube 7 is thus prevented.
In an embodiment, the casing 6 comprises at least a first casing portion and a second casing portion. The first casing portion and the second casing portion are articulated in order to enable relative movement of one casing portion relative to the other casing portion between:
- an open position for putting the bag 3 in place;
- a closed position draining the solution contained in the bag 3.

In the method for draining a solution contained in a bag 3 accommodated between a constant volume deformable cushion 4 and a variable volume bladder 5 of a device according one possible embodiment, the variable volume bladder 5 is deformed by a source of hydraulic power feeding said bladder 5 with hydraulic fluid, pressing the bag 3 against the deformable cushion 4 and causing the solution to empty out of the bag 3.

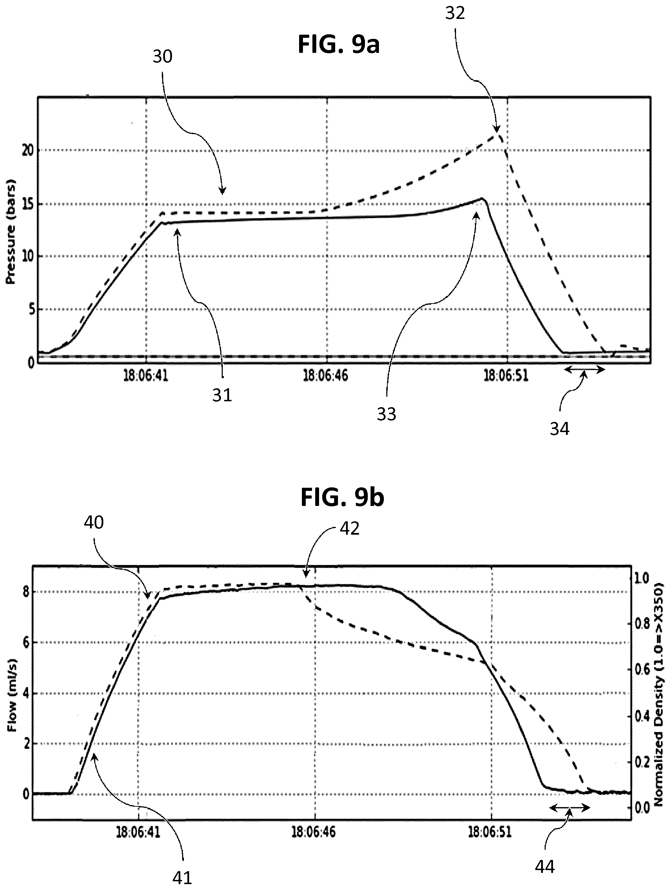

Figure 9a and 9b show test results comparing:
- Embodiment: draining a bag with a spacer member as disclosed in WO 2015/121355 with a device according to an embodiment of the invention corresponding to the embodiment shown on Figures 6 and 8a-8b;
- Prior art: draining a bag with a spacer member as disclosed in WO 2015/121355 with a device as disclosed in WO 2013/1862289, i.e. without the elevated part 22.

The test parameters are as follows:
- Solution: Xenetix® 350 (lobitridol 76.78%);
- Flow rate : 8 mL/s.
Other parameters are identical for the two tests.

Figure 9a shows the evolution of the pressure (in bars) at the output of the tube 7. The dashed curve 30 represents the pressure of the prior art, the solid curve 31 represents the pressure of the tested embodiment. The end of injection with a prior art device used to show a substantial peak 32 of pressure. The end of the draining with the new device now shows a much lower peak 33 of pressure. In addition, the peak 32 of pressure starts much earlier than the lower peak 33 of pressure. The invention allows a much more stable pressure. Furthermore, by the use of a prior art device, the end of the injection is substantially delayed and occurs with a delay 34 with respect to the tested embodiment, as shown on figure 9a. This could lead to a failure of the X-ray examination. The new device allows to be really closer to the estimation that is given by the injector to the radiologist before the examination.

Figure 9b shows the evolution of the flow rate (in mL/s) at the output of the tube 7 corresponding to the draining of Figure 9a. The dashed curve 40 represents the flow rate with the prior art device, the solid curve 41 represents the flow rate of the tested embodiment. The flow rate 41 with the prior art device shows a very early decrease 42, corresponding to the beginning of the pressure peak 32. As a consequence, draining the bag 3 with the prior art device requires a long time. The flow rate 41 with the tested embodiment is kept stable even after the flow rate 40 of the prior art device began to decrease. The time for emptying the bag 3 with the new device according to the invention will be really close to the time estimation given by the injector (before injection) whereas time of emptying the bag 3 could be greatly delayed with prior art device, showing a delay 44 for the end of the injection. Radiologist are using this estimated time of the injection in order to synchronize the time when the scanner is starting to make the data acquisition.

These results show that beyond avoiding collapsing of the walls of the bag, the invention allows to respect the estimated injection time by a more stable flow rate and pressure when draining the bag 3. This provides the radiologist with a much more reliable information and avoids him to fail the procedure.

While the present invention has been described with respect to certain preferred embodiments, it is obvious that it is in no way limited thereto and it comprises all the technical equivalents of the means described and their combinations. In particular, it will be apparent to those skilled in the art that various changes and modifications may be made without departing from the scope of the invention as defined in the appended claims.

## Claims

1. A device for draining a bag (3) containing a solution, the device comprising a casing (6) accommodating at least a deformable cushion (4) and a variable volume bladder (5), the device being configured to accommodate the bag (3) between said constant volume deformable cushion (4) and said variable volume bladder (5),
wherein said casing (6) comprises a first casing portion (14) defining a cavity (15) having a depth, said deformable cushion (4) being arranged on a surface of said cavity (15) over a cushion thickness, said first casing portion (14) comprising an output side (18) configured to accommodate an output tube (7) of the bag (3),
**characterized in that**:
the first casing portion (14) comprises an elevated part (22) protruding from the cavity (15) and extending from the output side (18) towards an opposite side (19) of the cavity, the deformable cushion (4) covering the cavity and the elevated part (22), said elevated part defining a support area for the bag.

2. The device according to any one of the preceding claims, wherein the elevated part (22) extends over at least one tenth of a distance between the output side (18) and the opposite side (19) of the cavity.

3. The device according to any one of the preceding claims, wherein the elevated part (22) extends over at least 2 cm from the output side (18) towards the opposite side (19) of the cavity.

4. The device according to any one of the preceding claims, wherein the elevated part (22) protrudes from a deepest part (16) of the cavity of more than half the cavity depth at the deepest part.

5. The device according to any one of the preceding claims, wherein the deformable cushion (4) is arranged on the surface of the cavity (15) and of the elevated part (22).

6. The device according to any one of the preceding claims, wherein the cushion thickness on the elevated part (22) is less than 8 mm.

7. The device according to any one of the preceding claims, wherein the cushion thickness over a deepest part of the cavity is more than 1 cm.

8. The device according to any one of the preceding claims, wherein the deformable cushion (4) fills the cavity (15) and in the absence of a bag defines a plane covering said cavity (15) and the elevated part (22).

9. The device according to any one of the preceding claims, wherein the deformable cushion (4) is made of silicone.

10. The device according to any one of the preceding claims, wherein the casing (6) comprises at least a second casing portion, the first casing portion (14) and said second casing portion being articulated in order to enable relative movement of one casing portion relative to the other casing portion between:
- an open position for putting the bag (3) in place;
- a closed position for draining the solution contained in the bag.

11. A method for draining a solution contained in a bag (3) accommodated between a constant volume deformable cushion (4) and a variable volume bladder (5) of a device according to any one of the preceding claims, wherein the variable volume bladder (5) is deformed by a source of hydraulic power feeding said bladder with hydraulic fluid, pressing the bag (3) against the deformable cushion (4) and causing solution to empty out of the bag (3).
